# EUROPEAN PATENT SPECIFICATION

(11) **EP 0 511 800 B1**
(45) Date of publication and mention of the grant of the patent: **23.10.1996**
(21) Application number: 92303753.5
(22) Date of filing: 27.04.1992
(51) Int. Cl.: A61K 31/71, A61K 9/24, A61K 9/28

(54) **Pharmaceutical formulation containing dirithromycin**
Dirithromycin enthaltendes Arzneimittel
Composition pharmaceutique à base de dirithromycine

(30) Priority: 29.04.1991 US 692842
(43) Date of publication of application: 04.11.1992
(73) Proprietor: ELI LILLY AND COMPANY, Indianapolis, Indiana 46285 (US)
(72) Inventor: Greene, James Michael, Indianapolis, Indiana 46217 (US); Hankins, Holly Marie, Indianapolis, Indiana 46231 (US); Stephenson, Gregory Alan, West Lafayette, Indiana 47906 (US); Wirth, David Dale, Lafayette, Indiana 47905 (US)
(74) Representative: Hudson, Christopher Mark

(56) References cited:
- EP-A- 0 208 971
- DE-A- 2 515 075

## Description

This invention relates to a pharmaceutical formulation, more particularly to a tablet containing a novel polymorph of a macrolide antibiotic.

Dirithromycin is a macrolide antibiotic derived from erythromycin. The antibiotic is described in U.S. Patent No. 4,048,306 and has the chemical name, Erythromycin, 9-deoxo-11-deoxy-9,11-[imino[2-(2-methoxyethoxy)ethylidene]oxy]-, [9S,16R]. Dirithromycin prepared by the method described in U.S. Patent No. 4,048,306 is produced in the form of a polymorph which is hereinafter referred to as "Form I". It has the following x-ray powder diffraction pattern, wherein D represents the interplanar spacing and I/Iₒ the relative intensity:

| D(Å) | I/Iₒ |
|---|---|
| 11.28 | 1.00 |
| 9.81 | 0.35 |
| 8.53 | 0.76 |
| 7.67 | 0.23 |
| 7.12 | 0.02 |
| 6.94 | 0.02 |
| 6.66 | 0.10 |
| 6.39 | 0.09 |
| 5.97 | 0.21 |
| 5.65 | 0.69 |
| 5.42 | 0.67 |
| 5.18 | 0.23 |
| 4.98 | 0.08 |
| 4.83 | 0.31 |
| 4.64 | 0.07 |
| 4.43 | 0.40 |
| 4.26 | 0.17 |
| 4.14 | 0.05 |
| 4.06 | 0.15 |
| 3.86 | 0.15 |
| 3.76 | 0.17 |
| 3.62 | 0.10 |
| 3.50 | 0.08 |
| 3.43 | 0.03 |
| 3.35 | 0.07 |
| 3.04 | 0.07 |
| 2.95 | 0.02 |
| 2.88 | 0.02 |
| 2.84 | 0.02 |
| 2.71 | 0.03 |
| 2.66 | 0.02 |
| 2.58 | 0.03 |

The Applicants have now discovered that Form I is metastable and is therefore not well suited for use in pharmaceutical formulations such as tablets.

However, surprisingly, and in accordance with the invention, it has now been discovered that a second polymorph of dirithromycin, hereinafter referred to as "Form II", is stable, and therefore is well adapted for use in pharmaceutical formulations such as tablets.

Thus, in one embodiment of the invention, there is provided a tablet containing dirithromycin Form II as an active ingredient. The tablet will also, of course, contain one or more pharmaceutically-acceptable excipients, such as binders, lubricants, disintegrants, fillers, etc.

This new polymorph has essentially the following x-ray powder diffraction pattern, wherein D represents the interplanar spacing and I/Iₒ the relative intensity:

| D(Å) | I/Iₒ |
|---|---|
| 14.17 | 0.02 |
| 11.96 | 0.27 |
| 10.43 | 0.11 |
| 9.65 | 1.00 |
| 8.86 | 0.84 |
| 8.18 | 0.54 |
| 7.07 | 0.33 |
| 6.99 | 0.10 |
| 6.84 | 0.21 |
| 6.59 | 0.03 |
| 6.24 | 0.05 |
| 6.07 | 0.29 |
| 5.97 | 0.19 |
| 5.77 | 0.06 |
| 5.54 | 0.36 |
| 5.50 | 0.47 |
| 5.45 | 0.26 |
| 5.13 | 0.22 |
| 5.11 | 0.29 |
| 4.84 | 0.29 |
| 4.75 | 0.47 |
| 4.72 | 0.42 |
| 4.50 | 0.62 |
| 4.44 | 0.31 |
| 4.24 | 0.20 |
| 4.20 | 0.05 |
| 4.11 | 0.17 |
| 4.09 | 0.18 |
| 3.92 | 0.14 |
| 3.87 | 0.12 |
| 3.83 | 0.12 |
| 3.73 | 0.06 |
| 3.55 | 0.08 |
| 3.49 | 0.15 |
| 3.46 | 0.07 |
| 3.42 | 0.11 |
| 3.33 | 0.05 |
| 3.17 | 0.04 |
| 3.11 | 0.02 |
| 2.96 | 0.04 |
| 2.83 | 0.02 |
| 2.74 | 0.04 |
| 2.57 | 0.03 |

The above x-ray powder diffraction patterns were obtained using a Nicolet 12V powder diffractometer equipped with a graphite monochrometer with copper radiation of 1 = 1.5418/angstroms. Those skilled in the art will appreciate that the intensity values are influenced by factors such as sample preparation and instrument variations. The D-spacings therefore constitute the essence of the diffraction pattern.

Since the active ingredient of the tablet (Form II) is to be used as a pharmaceutical it should be in a pharmaceutical state of purity. For stability purposes, Form II should not contain substantial amounts of Form I. Preferably, Form II should be associated with less than 10% of Form I, most preferably less than 5%.

Dirithromycin is a very effective antibiotic and has considerable advantages over other macrolide antibiotics since it need only be administered once a day in a total dosage of up to 500 mg. However, some care is needed in the design of orally acceptable formulations since the compound is sensitive to acidic media such as present in the stomach. In this context, reference is made to U.S. Patent No. 4,755,385, and it is preferred that the tablet of the invention be coated by a layer resistant to gastric juices, i.e. an "enteric coating". The nature of such coatings are well known to those skilled in the art. Frequently, a lacquer is used to form the coating.

The following Examples 1 to 4 illustrate the preparation of Form II.

### Example 1

### Isolation of Dirithromycin Form II From the Acetonate

A 20 g portion of non-solvated dirithromycin was added to a solvent mixture comprised of 81 ml acetone and 9 ml water. The solution was heated to about 60°C. The solution was maintained at 60°C until the reaction volume was 35 ml. An additional 100 ml portion of water was added slowly over 1 hour. The mixture was allowed to cool to room temperature. The cooled mixture was placed in an ice bath and stirred for 1 hour. The solid was isolated by filtration, and rinsed with 25 ml of chilled solvent comprised of 67% water and 33% acetone. The solid was rinsed with a 40 ml portion of water at ambient temperature. The wet cake of acetone solvate of dirithromycin was left at ambient conditions overnight.

A 180 ml portion of water was added to the wet cake of acetone solvate. The mixture was heated to 70°C, and was stirred at 70°C with a nitrogen purge for 4 hours. The solid was immediately isolated by filtration and rinsed with a 30 ml portion of water which had been warmed to 70°C. The isolated solid was dried in vacuo at 40°C overnight. The solid was identified as Form II dirithromycin by x-ray powder diffractometry.
Total yield: 90.8%

### Example 2

### Isolation of Form II Dirithromycin

A 10.0 g sample of acetone solvate of dirithromycin was added with stirring to 100 ml of water with nitrogen purge. The temperature of the reaction mixture was increased to 74°C and stirred at about 72-75°C for 4 hours. The mixture was vacuum filtered and washed with about 35 ml of 60°C water. The solid was dried in vacuo at 50°C overnight. The solid was identified as Form II dirithromycin, by x-ray powder diffractometry. The total yield of the reaction was 8.74 g (87.4%).

### Example 3

### Isolation of Form II Dirithromycin

A 15 g portion of Form I dirithromycin was added to 150 ml water. The reaction mixture was heated to 74°C and stirred for 4 hours at 74°C. The solid was isolated by filtration and washed with two washes, each of 40 ml of 70°C water. The sample was dried in a vacuum oven at 25°C for about 68 hours. The product was identified as Form II dirithromycin by x-ray powder diffractometry.
Yield: 97.5%

### Example 4

### Isolation of Form II Dirithromycin

A 3.01 g portion of Form I dirithromycin was added to 15 ml ethyl acetate. The reaction mixture was heated to about 76°C and the mixture was allowed to boil until the reaction volume was about 10 ml. A 20 ml portion of n-octane was added. The mixture was allowed to cool to room temperature. The solid was isolated by filtration. The sample was dried at room temperature. The product was identified as Form II dirithromycin by x-ray powder diffractometry.
Total Yield: 95%

General methods of preparing pharmaceutical formulations containing dirithromycin are disclosed in U.S. Patent No. 4,755,385. In substance, the process of manufacture involves the admixture of Form II with the necessary pharmaceutically acceptable carriers followed by compression into tablets and, optionally, coating with a layer resistant to gastric juices. However, the following specific Example illustrates the preparation of a coated tablet in accordance with the invention.

### Example 5

The following data relates to a tablet containing 250 mg of dirithromycin Form II as the active ingredient.

| Core Tablet | MG/Tablet |
|---|---|
| Dirithromycin Form II | 250.0 mg |
| Magnesium Carbonate | 250.0 mg |
| Microcrystalline Cellulose | 199.6 mg |
| Sodium Starch Glycolate | 10.0 mg |
| Hydroxypropyl Cellulose | 15.0 mg |
| Croscarmellose Sodium | 10.0 mg |
| Magnesium Stearate | 12.0 mg |

| Subcoating | |
|---|---|
| Hydroxypropyl Methylcellulose 2910 (E-5) | 16.21 mg |
| Polyethylene Glycol (3350) | 4.63 mg |
| Propylene Glycol | 6.95 mg |
| Benzyl Alcohol | 2.31 mg |

| Enteric Coating | |
|---|---|
| Methacrylic Acid Copolymer Aqueous | |
| Dispersion (L30D) (Solids) | 48.65 mg |
| Color Mixture White T3166-WE (Solids) | 20.31 mg |
| Triethyl Citrate | 1.65 mg |
| Talc | Trace |

### Core Tablets:

The lots may be prepared as a single entity or fraction thereof and all units blended as a dry mix before compression. All ingredients are security sieved. Dirithromycin (Form II), magnesium carbonate, microcrystalline cellulose, sodium starch glycolate, hydroxypropyl cellulose, and magnesium stearate are thoroughly blended in a ribbon mixer or other type of suitable mixer such as a V-blender, conical mixer, etc. The resulting powder mixture is compacted using a roller compactor or equivalent dry granulating equipment. The granulation is sized if necessary using an oscillating granulator, fitz mill or other suitable milling equipment. The sized granulation is blended with additional microcrystalline cellulose, croscarmellose sodium, and magnesium stearate in a ribbon mixer or other type of suitable mixer such as a V-blender, conical mixer, etc. After blending, the weight is checked against theoretical. The granulation is compressed on a conventional type compression machine such as a Manesty Betapress, etc. weight checks of the core tablets are made routinely throughout the compression process and the samples are pooled for testing.

### Subcoating:

The subcoating is prepared by dissolving hydroxypropyl methylcellulose 2910 (E-5), polyethylene glycol, propylene glycol and benzyl alcohol in purified water. This coating solution can be prepared using a Groen kettle, stockpot, or equivalent and a means to provide agitation such as a Lightening mixer or equivalent.

This coating solution is applied to the core tablets from above in an Accela Cota coating pan or equivalent film coating equipment. Suitable spraying systems such as the Accela-Spray II, Nordson spray unit, Graco spray unit or equivalent spraying equipment are utilized. Alternate spraying and drying cycles are used if necessary until the desired amount of coating solution has been applied.

### Enteric Coating:

The enteric coating suspension is prepared by mixing methacrylic acid copolymer aqueous dispersion (L30D), color mixture white T3166-WE, and triethyl citrate with purified water. This coating suspension can be prepared using a Groen kettle, stockpot, or equivalent and a means to provide agitation such as a Lightning mixer or equivalent.

This coating suspension is applied to the subcoated tablets from above in an Accela Cota coating pan or equivalent film coating equipment. Suitable spraying systems such as the Accela-Spray II, Nordson spray unit, Graco spray unit or equivalent spraying equipment are utilized. Alternate spraying and drying cycles are used if necessary until the desired amount of coating suspension has been applied. The tablets may be finished by dusting lightly with talc (to increase the luster of the coated tablets). Only trace amounts of this polishing agent adheres to the finished tablets.

## Claims (Claims for the following Contracting State(s): AT, BE, CH, DE, DK, FR, GB, IT, LI, LU, NL, PT, SE)

1. A tablet containing as an active ingredient dirithromycin Form II, associated with one or more pharmaceutically acceptable excipients, said dirithromycin Form II having essentially the following x-ray powder diffraction pattern, wherein D represents the interplanar spacing and I/Iₒ the relative intensity:
| D(Å) | I/Iₒ |
|---|---|
| 14.17 | 0.02 |
| 11.96 | 0.27 |
| 10.43 | 0.11 |
| 9.65 | 1.00 |
| 8.86 | 0.84 |
| 8.18 | 0.54 |
| 7.07 | 0.33 |
| 6.99 | 0.10 |
| 6.84 | 0.21 |
| 6.59 | 0.03 |
| 6.24 | 0.05 |
| 6.07 | 0.29 |
| 5.97 | 0.19 |
| 5.77 | 0.06 |
| 5.54 | 0.36 |
| 5.50 | 0.47 |
| 5.45 | 0.26 |
| 5.13 | 0.22 |
| 5.11 | 0.29 |
| 4.84 | 0.29 |
| 4.75 | 0.47 |
| 4.72 | 0.42 |
| 4.50 | 0.62 |
| 4.44 | 0.31 |
| 4.24 | 0.20 |
| 4.20 | 0.05 |
| 4.11 | 0.17 |
| 4.09 | 0.18 |
| 3.92 | 0.14 |
| 3.87 | 0.12 |
| 3.83 | 0.12 |
| 3.73 | 0.06 |
| 3.55 | 0.08 |
| 3.49 | 0.15 |
| 3.46 | 0.07 |
| 3.42 | 0.11 |
| 3.33 | 0.05 |
| 3.17 | 0.04 |
| 3.11 | 0.02 |
| 2.96 | 0.04 |
| 2.83 | 0.02 |
| 2.74 | 0.04 |
| 2.57 | 0.03 |

2. A tablet as claimed in claim 1 coated with one or more layers resistant to gastric juices.

3. A pharmaceutical formulation containing as an active ingredient dirithromycin Form II as defined in claim 1, associated with one or more pharmaceutically acceptable excipients.

## Claims (Claims for the following Contracting State(s): ES, GR)

1. A process for producing a tablet which comprises mixing dirithromycin Form II with one or more pharmaceutically acceptable excipients, and forming a tablet from the mixture, said dirithromycin Form II having essentially the following x-ray powder diffraction pattern, wherein D represents the interplanar spacing and I/Iₒ the relative intensity:
| D(Å) | I/Iₒ |
|---|---|
| 14.17 | 0.02 |
| 11.96 | 0.27 |
| 10.43 | 0.11 |
| 9.65 | 1.00 |
| 8.86 | 0.84 |
| 8.18 | 0.54 |
| 7.07 | 0.33 |
| 6.99 | 0.10 |
| 6.84 | 0.21 |
| 6.59 | 0.03 |
| 6.24 | 0.05 |
| 6.07 | 0.29 |
| 5.97 | 0.19 |
| 5.77 | 0.06 |
| 5.54 | 0.36 |
| 5.50 | 0.47 |
| 5.45 | 0.26 |
| 5.13 | 0.22 |
| 5.11 | 0.29 |
| 4.84 | 0.29 |
| 4.75 | 0.47 |
| 4.72 | 0.42 |
| 4.50 | 0.62 |
| 4.44 | 0.31 |
| 4.24 | 0.20 |
| 4.20 | 0.05 |
| 4.11 | 0.17 |
| 4.09 | 0.18 |
| 3.92 | 0.14 |
| 3.87 | 0.12 |
| 3.83 | 0.12 |
| 3.73 | 0.06 |
| 3.55 | 0.08 |
| 3.49 | 0.15 |
| 3.46 | 0.07 |
| 3.42 | 0.11 |
| 3.33 | 0.05 |
| 3.17 | 0.04 |
| 3.11 | 0.02 |
| 2.96 | 0.04 |
| 2.83 | 0.02 |
| 2.74 | 0.04 |
| 2.57 | 0.03 |

2. A process according to claim 1 which comprises coating the tablet with one or more layers resistant to gastric juices.

3. A tablet containing as an active ingredient dirithromycin Form II, as defined in claim 1, associated with one or more pharmaceutically acceptable excipients.

4. A tablet as claimed in claim 3 coated with one or more layers resistant to gastric juices.

## Patentansprüche (Patentansprüche für folgende(n) Vertragsstaat(en): AT, BE, CH, DE, DK, FR, GB, IT, LI, LU, NL, PT, SE)

1. Tablette, die als Wirkstoff Dirithromycin Form II zusammen mit einem oder mehreren pharmazeutisch annehmbaren Hilfsstoffen enthält, wobei dieses Dirithromycin Form II im wesentlichen das folgende Röntgendiffraktionsmuster am Pulver aufweist, worin D für den Interplanarabstand und I/I₀ für die relative Intensität stehen:
| D(Å) | I/I₀ |
|---|---|
| 14,17 | 0,02 |
| 11,96 | 0,27 |
| 10,43 | 0,11 |
| 9,65 | 1,00 |
| 8,86 | 0,84 |
| 8,18 | 0,54 |
| 7,07 | 0,33 |
| 6,99 | 0,10 |
| 6,84 | 0,21 |
| 6,59 | 0,03 |
| 6,24 | 0,05 |
| 6,07 | 0,29 |
| 5,97 | 0,19 |
| 5,77 | 0,06 |
| 5,54 | 0,36 |
| 5,50 | 0,47 |
| 5,45 | 0,26 |
| 5,13 | 0,22 |
| 5,11 | 0,29 |
| 4,84 | 0,29 |
| 4,75 | 0,47 |
| 4,72 | 0,42 |
| 4,50 | 0,62 |
| 4,44 | 0,31 |
| 4,24 | 0,20 |
| 4,20 | 0,05 |
| 4,11 | 0,17 |
| 4,09 | 0,18 |
| 3,92 | 0,14 |
| 3,87 | 0,12 |
| 3,83 | 0,12 |
| 3,73 | 0,06 |
| 3,55 | 0,08 |
| 3,49 | 0,15 |
| 3,46 | 0,07 |
| 3,42 | 0,11 |
| 3,33 | 0,05 |
| 3,17 | 0,04 |
| 3,11 | 0,02 |
| 2,96 | 0,04 |
| 2,83 | 0,02 |
| 2,74 | 0,04 |
| 2,57 | 0,03 |

2. Tablette nach Anspruch 1, die mit einer oder mehreren Schichten ummantelt ist, welche gegenüber Magensäften resistent sind.

3. Pharmazeutische Formulierung, die als Wirkstoff Dirithromycin Form II nach Anspruch 1 zusammen mit einem oder mehreren pharmazeutisch annehmbaren Hilfsstoffen enthält.

## Patentansprüche (Patentansprüche für folgende(n) Vertragsstaat(en): ES, GR)

1. Verfahren zur Herstellung einer Tablette, gekennzeichnet durch Mischen von Dirithromycin Form II zusammen mit einem oder mehreren pharmazeutisch annehmbaren Hilfsstoffen, und Bilden einer Tablette aus dem Gemisch, wobei dieses Dirithromycin Form II im wesentlichen das folgende Röntgendiffraktionsmuster am Pulver aufweist, worin D für den Interplanarabstand und I/I₀ für die relative Intensität stehen:
| D(Å) | I/I₀ |
|---|---|
| 14,17 | 0,02 |
| 11,96 | 0,27 |
| 10,43 | 0,11 |
| 9,65 | 1,00 |
| 8,86 | 0,84 |
| 8,18 | 0,54 |
| 7,07 | 0,33 |
| 6,99 | 0,10 |
| 6,84 | 0,21 |
| 6,59 | 0,03 |
| 6,24 | 0,05 |
| 6,07 | 0,29 |
| 5,97 | 0,19 |
| 5,77 | 0,06 |
| 5,54 | 0,36 |
| 5,50 | 0,47 |
| 5,45 | 0,26 |
| 5,13 | 0,22 |
| 5,11 | 0,29 |
| 4,84 | 0,29 |
| 4,75 | 0,47 |
| 4,72 | 0,42 |
| 4,50 | 0,62 |
| 4,44 | 0,31 |
| 4,24 | 0,20 |
| 4,20 | 0,05 |
| 4,11 | 0,17 |
| 4,09 | 0,18 |
| 3,92 | 0,14 |
| 3,87 | 0,12 |
| 3,83 | 0,12 |
| 3,73 | 0,06 |
| 3,55 | 0,08 |
| 3,49 | 0,15 |
| 3,46 | 0,07 |
| 3,42 | 0,11 |
| 3,33 | 0,05 |
| 3,17 | 0,04 |
| 3,11 | 0,02 |
| 2,96 | 0,04 |
| 2,83 | 0,02 |
| 2,74 | 0,04 |
| 2,57 | 0,03 |

2. Verfahren nach Anspruch 1, gekennzeichnet durch Ummanteln der Tablette mit einer oder mehreren Schichten, die gegenüber Magensäften resistent sind.

3. Tablette, die als Wirkstoff Dirithromycin Form II nach Anspruch 1 zusammen mit einem oder mehreren pharmazeutisch annehmbaren Hilfsstoffen enthält.

4. Tablette nach Anspruch 3, die mit einer oder mehreren Schichten ummantelt ist, welche gegenüber Magensäften resistent sind.

## Revendications (Revendications pour l'(les) Etat(s) contractant(s) suivant(s): AT, BE, CH, DE, DK, FR, GB, IT, LI, LU, NL, PT, SE)

1. Comprimé contenant, comme ingrédient actif, de la dirithromycine de Forme II, en association avec un ou plusieurs excipients pharmaceutiquement acceptables, ladite dirithromycine de Forme II possédant essentiellement le motif de diffraction de rayons X de la poudre ci-après, où D représente l'écartement des plans et I/I₀ représente l'intensité relative:
| D(Å) | I/I₀ |
|---|---|
| 14,17 | 0,02 |
| 11,96 | 0,27 |
| 10,43 | 0,11 |
| 9,65 | 1,00 |
| 8,86 | 0,84 |
| 8,18 | 0,54 |
| 7,07 | 0,33 |
| 6,99 | 0,10 |
| 6,84 | 0,21 |
| 6,59 | 0,03 |
| 6,24 | 0,05 |
| 6,07 | 0,29 |
| 5,97 | 0,19 |
| 5,77 | 0,06 |
| 5,54 | 0,36 |
| 5,50 | 0,47 |
| 5,45 | 0,26 |
| 5,13 | 0,22 |
| 5,11 | 0,29 |
| 4,84 | 0,29 |
| 4,75 | 0,47 |
| 4,72 | 0,42 |
| 4,50 | 0,62 |
| 4,44 | 0,31 |
| 4,24 | 0,20 |
| 4,20 | 0,05 |
| 4,11 | 0,17 |
| 4,09 | 0,18 |
| 3,92 | 0,14 |
| 3,87 | 0,12 |
| 3,83 | 0,12 |
| 3,73 | 0,06 |
| 3,55 | 0,08 |
| 3,49 | 0,15 |
| 3,46 | 0,07 |
| 3,42 | 0,11 |
| 3,33 | 0,05 |
| 3,17 | 0,04 |
| 3,11 | 0,02 |
| 2,96 | 0,04 |
| 2,83 | 0,02 |
| 2,74 | 0,04 |
| 2,57 | 0,03 |

2. Comprimé selon la revendication 1, enrobé avec une ou plusieurs couches résistant aux sucs gastriques.

3. Composition pharmaceutique contenant, comme ingrédient actif, de la dirithromycine de Forme II telle que définie à la revendication 1, en association avec un ou plusieurs excipients pharmaceutiquement acceptables.

## Revendications (Revendications pour l'(les) Etat(s) contractant(s) suivant(s): ES, GR)

1. Procédé pour préparer un comprimé qui comprend le fait de mélanger de la dirithromycine de Forme II avec un ou plusieurs excipients pharmaceutiquement acceptables et le fait de former un comprimé à partir du mélange, ladite dirithromycine de Forme II possédant essentiellement le motif de diffraction de rayons X de la poudre ci-après, où D représente l'écartement des plans et I/I₀ représente l'intensité relative:
| D(Å) | I/I₀ |
|---|---|
| 14,17 | 0,02 |
| 11,96 | 0,27 |
| 10,43 | 0,11 |
| 9,65 | 1,00 |
| 8,86 | 0,84 |
| 8,18 | 0,54 |
| 7,07 | 0,33 |
| 6,99 | 0,10 |
| 6,84 | 0,21 |
| 6,59 | 0,03 |
| 6,24 | 0,05 |
| 6,07 | 0,29 |
| 5,97 | 0,19 |
| 5,77 | 0,06 |
| 5,54 | 0,36 |
| 5,50 | 0,47 |
| 5,45 | 0,26 |
| 5,13 | 0,22 |
| 5,11 | 0,29 |
| 4,84 | 0,29 |
| 4,75 | 0,47 |
| 4,72 | 0,42 |
| 4,50 | 0,62 |
| 4,44 | 0,31 |
| 4,24 | 0,20 |
| 4,20 | 0,05 |
| 4,11 | 0,17 |
| 4,09 | 0,18 |
| 3,92 | 0,14 |
| 3,87 | 0,12 |
| 3,83 | 0,12 |
| 3,73 | 0,06 |
| 3,55 | 0,08 |
| 3,49 | 0,15 |
| 3,46 | 0,07 |
| 3,42 | 0,11 |
| 3,33 | 0,05 |
| 3,17 | 0,04 |
| 3,11 | 0,02 |
| 2,96 | 0,04 |
| 2,83 | 0,02 |
| 2,74 | 0,04 |
| 2,57 | 0,03 |

2. Procédé selon la revendication 1, qui comprend le fait d'enrober le comprimé avec une ou plusieurs couches résistant aux sucs gastriques.

3. Comprimé contenant, comme ingrédient actif, de la dirithromycine de Forme II telle que définie à la revendication 1, en association avec un ou plusieurs excipients pharmaceutiquement acceptables.

4. Comprimé selon la revendication 3, enrobé avec une ou plusieurs couches résistant aux sucs gastriques.
